(19) Europäisches Patentamt
European Patent Office
Office européen des brevets

(11) **EP 2 389 139 B1**

(12) **EUROPÄISCHE PATENTSCHRIFT**

(45) Veröffentlichungstag und Bekanntmachung des Hinweises auf die Patenterteilung:
**01.05.2013 Patentblatt 2013/18**

(21) Anmeldenummer: **10704733.4**

(22) Anmeldetag: **21.01.2010**

(51) Int Cl.:
*A61F 2/16* (2006.01)     *G02C 7/06* (2006.01)

(86) Internationale Anmeldenummer:
**PCT/AT2010/000019**

(87) Internationale Veröffentlichungsnummer:
**WO 2010/083546 (29.07.2010 Gazette 2010/30)**

(54) **LINSE MIT DISKRETEM ZIRKULÄREM BRECHKRAFTPROFIL**

LENS HAVING DISCRETE CIRCULAR REFRACTIVE POWER PROFILE

LENTILLE PRÉSENTANT UN PROFIL DE PUISSANCE CIRCULAIRE DISCRET

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **21.01.2009 AT 962009**

(43) Veröffentlichungstag der Anmeldung:
**30.11.2011 Patentblatt 2011/48**

(73) Patentinhaber: **Fiala, Werner**
**1180 Wien (AT)**

(72) Erfinder: **Fiala, Werner**
**1180 Wien (AT)**

(74) Vertreter: **Weiser, Andreas**
**Patentanwalt**
**Kopfgasse 7**
**1130 Wien (AT)**

(56) Entgegenhaltungen:
WO-A1-03/009053          WO-A1-2006/047698
DE-A1-102005 028 933     US-A- 5 796 462

• FIALA W ET AL: "ANALYTICAL APPROACH TO DIFFRACTIVE MULTIFOCAL LENSES" EUROPEAN PHYSICAL JOURNAL APPLIED PHYSICS, EDP SCIENCES, LES ULIS, FR LNKD-DOI:10.1051/EPJAP:2000108, Bd. 9, Nr. 3, 1. März 2000 (2000-03-01), Seiten 227-234, XP000963120 ISSN: 1286-0042 in der Anmeldung erwähnt

**Beschreibung**

**Einleitung und Stand der Technik**

Die vorliegende Erfindung bezieht sich auf eine Linse mit zirkulärem Brechkraftprofil.

Im Gegensatz zu rotationssymmetrischen Linse weisen Linsen mit zirkulärem Brechkraftprofil in verschiedenen Meridianen verschiedene Brechkräfte auf. Gegenwärtig sind nur solche zirkuläre Brechkraftprofile bekannt, welche sog. torische Linsen ergeben.

Torische Linsen besitzen in zwei Linsenmeridianen, den sogenannten Hauptmeridianen, zwei verschiedene Brechkräfte. In der Regel stehen diese zwei Linsenmeridiane orthogonal zueinander. Die kleinere der beiden Brechkräfte wird im allgemeinen "Sphäre" genannt. Die Differenz zwischen der größeren und der kleineren der beiden Brechkräfte wird im allgemeinen "Zylinder" genannt. Dabei können die Meridiane in den Brechkräften "Sphäre" und "Sphäre plus Zylinder" kreisförmig oder auch nicht-kreisförmig ausgebildet sein, also z.B. durch die Funktion einer Asphäre beschrieben sein; in diesem Falle besitzen solche Flächen in verschiedenen Meridianen im allgemeinen neben den verschiedenen Radien auch verschiedene Asphärizitäten (WO 2006/136424 A1). Die Meridiane zwischen den Hauptmeridianen weisen Brechkräfte auf, die zwischen der kleineren und der größeren Brechkraft der Hauptmeridiane liegen.

Torische Linsen werden beispielsweise dazu verwendet, den ocularen Astigmatismus eines Auges zu kompensieren; dabei kann es sich um einen cornealen oder einen Linsenastigmatismus oder eine Kombination von beiden handeln. Torische Linsen werden aber auch zur Korrektur des in anderen optischen Systemen gegebenenfalls auftretenden Astigmatismus verwendet.

Der Astigmatismus stellt einen Wellenfrontfehler dar, der durch die Zernike-Polynome

$$Z(2,2) = \sqrt{6} \times R^2 \times \cos 2\phi \quad bzw. \quad Z(2,-2) = \sqrt{6} \times R^2 \times \sin 2\phi \tag{1}$$

charakterisiert werden kann, je nachdem, ob die "Sphäre" bei Null oder 90° eines Koordinatensystems gegeben ist.

Gemäß den obigen Polynomen wiederholt sich der Wellenfrontfehler alle 180°, da die Funktionen $\sin 2\phi$ bzw. $\cos 2\phi$ für $\phi$ und $\phi+180°$ identisch sind.

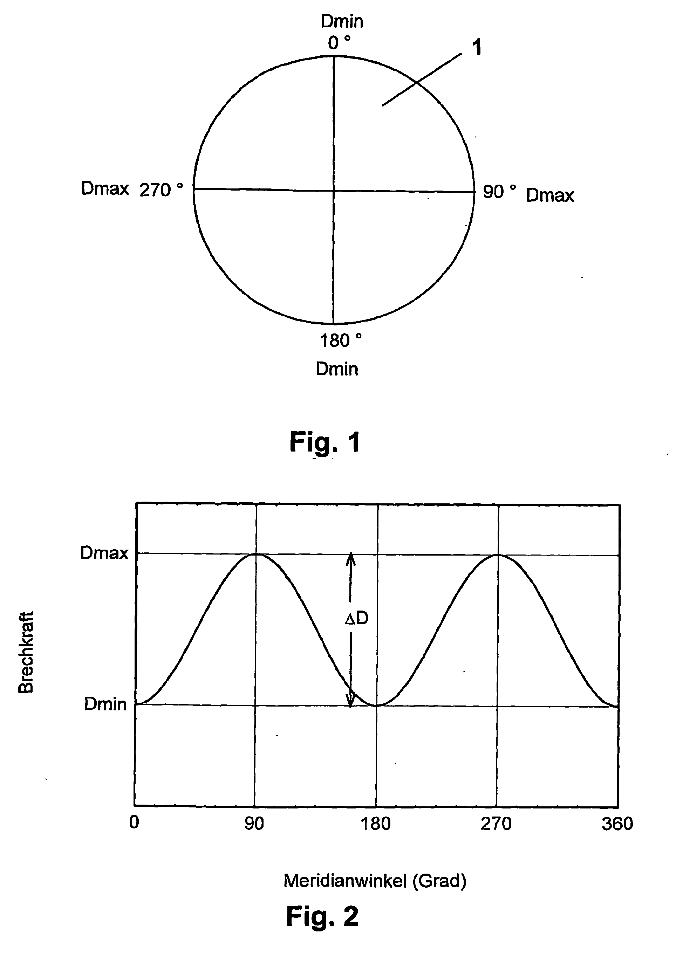

In Fig. 1 ist eine herkömmliche torische Linse in Aufsicht dargestellt. Die torische Linse kann aus einer Linsenfläche, die torisch ist, und einer rotationssymmetrischen Linsenfläche bestehen. Sie kann aber auch aus zwei torischen Linsenflächen bestehen ("bitorisch" gemäß WO 2006236424 A1, siehe oben). Besteht die torische Linse aus einer torischen Fläche und einer rotationssymmetrischen Fläche, dann wird der Unterschied zwischen den beiden Brechkräften in den Hauptmeridianen ausschließlich durch die torische Linsenfläche bewerkstelligt.

In Fig. 2 ist das entsprechende zirkuläre Brechkraftprofil der in Fig. 1 schematisch dargestellten Linse gezeigt.

Bei herkömmlichen torischen Linsen spannen die Normalvektoren auf die Linsenfläche in lediglich zwei Meridianen, den Hauptmeridianen, mit der Linsenachse Ebenen auf. Diese Meridiane zeichnen sich dadurch aus, daß in ihnen die Ableitung

$$\frac{\partial D}{\partial \alpha} = 0$$

ist, wobei D die Brechkraft ist und $\alpha$ der Meridianwinkel.

In sämtlichen anderen Meridianen sind die Normalvektoren auf die Linsenfläche zur Linsenachse windschief.

Dieser Sachverhalt bei herkömmlichen torischen bzw. bitorischen Linsen wird aus formalen Gründen nun dahingehend beschrieben, daß die Flächen solcher Linsen in lediglich vier Halbmeridianen Normalvektoren aufweisen, die mit der Linsenachse Ebenen aufspannen.

Der oculare Wellenfrontfehler Astigmatismus mit einem Zylinder im Ausmaß von bis zu einer Dioptrie wird oftmals nicht korrigiert, da ein mit diesem Wellenfrontfehler behaftetes Auge eine erhöhte Tiefenschärfe in der Größenordnung des Zylinders aufweist, und die durch den geringen Astigmatismus bedingte geringere Bildqualität zerebral kompensiert werden kann.

Auch in anderen optischen Systemen kann die Beeinträchtigung der Bildgebung durch eine astigmatische Wellenfront mit geringem Zylinder als akzeptabel gelten.

Neben dem Wellenfrontfehler Astigmatismus sind auch andere Wellenfrontfehler bekannt, so z.B. Dreiblattfehler (Trefoil), der mit den Zernike-Polynomen

$$Z(3,3) = \sqrt{8} \times R^3 \times \cos 3\phi \quad bzw. \quad Z(3,-3) = \sqrt{8} \times R^3 \times \sin 3\phi \qquad (2)$$

charakterisiert werden kann. Beim Dreiblattfehler wiederholt sich der Wellenfrontfehler alle 120°. Weiters gibt es die Wellenfrontfehler Vier-, Fünf-, Sechsblattfehler (Tetra-, Penta-, Hexafoil) etc. Allgemein können solche Mehrblattfehler durch Zernike-Polynome der folgenden Art beschrieben werden:

$$Z(n,m) = \sqrt{2(m+1)} \times R^n \times \cos m\phi \quad bzw. \quad Z(n,-m) = \sqrt{2(m+1)} \times R^n \times \sin m\phi$$

$$(3)$$

In den Ausdrücken (3) stellt m die Wiederholungsrate des Wellenfrontfehlers über 360° dar. Die Wiederholungsrate m drückt aus, bei welcher Drehung um 360°/m die Wellenfrontfläche mit der ursprünglichen Wellenfrontfläche gleich ist. Die Wiederholungsrate m ist bei Astigmatismus (Zweiblattfehler, "Bifoil") gleich 2, beim Dreiblattfehler (Trefoil) ist m = 3, beim Vierblattfehler (Tetrafoil) ist m = 4, usw. Die Zahl n im Polynom Z(n,m) stellt die größte Potenz des Einheitsradius R im Zernike-Polynom dar; sie ist für die gegenständlichen Überlegungen von untergeordneter Bedeutung.

Die Wiederholungsrate gemäß obiger Definition gilt nicht nur für Flächen von Wellenfrontfehlern, sondern auch für entsprechende nicht-rotationssymmetrische Flächen, wie z.B. Linsenflächen, im allgemeinen.

Mehrblattfehler zeichnen sich dadurch aus, daß die ganzen Zahlen n und m im Polynom Z(n, m) bzw. (Zn, -m) den gleichen Wert haben.

Daneben gibt es noch andere Wellenfrontfehler, die durch Zernike-Polynome Z(n, m) beschrieben werden können, bei denen n und m verschieden sind.

Herkömmliche torische Linsen können lediglich den Wellenfrontfehler Astigmatismus ("Zweiblattfehler", m = 2) kompensieren. Zur Korrektur von Wellenfrontfehlern, bei denen gemäß den Ausdrücken (3) die Wiederholungsrate m > 2 ist, sind keine Linsen bekannt.

Neben Wellenfrontfehlern mit Wiederholungsraten m ≥ 2 sind auch Wellenfrontfehler mit m = 1 bekannt, wie Verkippung Z(1,1) bzw. Z(1,-1) und Koma Z(2,1) bzw. Z(2,-1). Auch solche Wellenfrontfehler können weder mit herkömmlichen rotationssymmetrischen Linsen noch mit herkömmlichen torischen Linsen kompensiert werden.

## Kurze Beschreibung der Erfindung

Ein Ziel der Erfindung ist eine Linse mit zirkulärem Brechkraftprofil, die eine erhöhte Tiefenschärfe besitzt.

Dieses Ziel wird mit einer Linse mit zirkulärem Brechkraftprofil erreicht, die sich dadurch auszeichnet, daß sie in zumindest einem Halbmeridian, der zwischen Halbmeridianen mit der minimalen und der maximalen Brechkraft der Linse liegt, eine diskrete Brechkraft aufweist, die zwischen der minimalen und der maximalen Brechkraft der Linse liegt.

Linsen dieser Art werden im weiteren "diskret torisch" (wenn m = 2) bzw. "diskret supertorisch" (wenn m ≠ 2) genannt und besitzen im Vergleich zu bekannten torischen Linsen eine erhöhte Tiefenschärfe, wie später noch im Detail erläutert wird.

Bevorzugt hat die Linse nur einen Halbmeridian mit der minimalen Brechkraft und nur einen Halbmeridian mit der maximalen Brechkraft der Linse.

Alternativ hat die Linse bevorzugt mehr als zwei Halbmeridiane mit der minimalen Brechkraft und mehr als zwei Halbmeridiane mit der maximalen Brechkraft der Linse.

Eine diskret supertorische Linse mit einer bevorzugten Wiederholungsrate von m = 1 ist zur Kompensation von Verkippung bzw. Koma geeignet.

Eine diskret torische Linse mit einer bevorzugten Wiederholungsrate von m = 2 eignet sich besonders zur Kompensation von Astigmatismus.

Eine diskret supertorische Linse mit bevorzugten Wiederholungsraten von m ≥ 3 dient insbesondere zur Kompensation von Mehrblattfehlern.

Ein weiterer Gegenstand der Erfindung ist eine Linse mit erhöhter Tiefenschärfe, die aus einer diskret torischen oder diskret supertorischen Linsenfläche und einer rotationssymmetrischen Linsenfläche besteht, welche gemäß US 5,982,543 (Fiala) bzw. US 7,287,852 B2 (Fiala) annulare Zonen aufweist, zwischen denen sich optische Stufen befinden, die größer sind als die Kohärenzlänge von polychromatischem Licht.

Demgemäß besteht eine weitere bevorzugte Ausführungsform der erfindungsgemäßen Linse darin, daß sie zusätzlich mit einem radialen Brechkraftprofil versehen ist.

Bevorzugt ist das zirkuläre Brechkraftprofil durch Gestaltung der einen und das radiale Brechkraftprofil durch Gestaltung der anderen Oberfläche der Linse gebildet.

Besonders bevorzugt ist das radiale Brechkraftprofil in an sich bekannter Weise durch annulare Zonen mit dazwischenliegenden optischen Stufen gebildet.

Weitere Merkmale und Vorteile der Erfindung ergeben sich aus der nachfolgenden Beschreibung von bevorzugten Ausführungsbeispielen und Bezugnahme auf die begleitenden Zeichnungen.

**Kurze Beschreibung der Figuren**

Fig. 1 stellt schematisch eine herkömmliche torische Linse in Aufsicht dar.

Fig. 2 zeigt schematisch das zirkuläre Brechkraftprofil einer Linse gemäß Fig. 1.

Fig. 3 stellt eine supertorische Linse in Aufsicht dar. Die Wiederholungsrate beträgt bei dieser Linse m = 4.

Fig. 4 zeigt schematisch das zirkuläre Brechkraftprofil einer Linse gemäß Fig. 3.

Fig. 5 stellt eine erfindungsgemäße diskret torische Linse in Aufsicht dar.

Fig. 6 zeigt schematisch das zirkuläre Brechkraftprofil einer Linse entsprechend Fig. 5.

Fig. 7 stellt eine supertorische Linse in Aufsicht dar. Die Wiederholungsrate dieser Linse ist m = 3.

Fig. 8 zeigt schematisch das zirkuläre Brechkraftprofil der Linse gemäß Fig. 7.

Fig. 9 zeigt schematisch eine diskret supertorische Linse gemäß der gegenständlichen Erfindung in Aufsicht. Die Wiederholungsrate dieser Linse ist m = 3; die Linse weist zumindest eine Fläche auf, bei der in 18 Halbmeridianen die Normalvektoren auf die Linsenfläche mit der Linsenachse Ebenen aufspannen.

Fig. 10 zeigt schematisch das zirkuläre Brechkraftprofil der Linse gemäß Fig. 9.

Fig. 11 zeigt den Querschnitt einer erfindungsgemäßen Linse mit großer Tiefenschärfe.

Fig. 12 zeigt in Aufsicht eine supertorische Linse, bei der die Wiederholungsrate m = 1 ist.

Fig. 13 zeigt schematisch das zirkuläre Brechkraftprofil einer Linse gemäß Fig. 12.

Fig. 14 zeigt in Aufsicht eine diskret supertorische Linse gemäß der Erfindung, bei der die Wiederholungsrate m = 1 ist. Die Linse weist zumindest eine Fläche auf, bei der in 8 Halbmeridianen die Normalvektoren auf die Linsenfläche mit der Linsenachse Ebenen aufspannen.

Fig. 15 zeigt schematisch das zirkuläre Brechkraftprofil einer Linse gemäß Fig. 14.

**Ausführliche Beschreibung bevorzugter Ausführungsformen der Erfindung**

In Fig. 1 ist eine herkömmliche torische Linse 1 dargestellt. Die Linse weist im Hauptmeridian 0° (= Hauptmeridian 180°) die minimale Brechkraft Dmin auf, im zweiten Hauptmeridian 90° (= Hauptmeridian 270°) besitzt sie die Brechkraft Dmax. Üblicherweise wird die Brechkraft Dmin als "Sphäre" bezeichnet, die Brechkraft Dmax als "Sphäre plus Zylinder". Die zirkuläre Brechkraft D($\alpha$) ändert sich stetig von Dmin bis Dmax und ist beispielsweise durch die Funktion

$$D(\alpha) = D\min \times \cos^2(\alpha) + D\max \times \sin^2(\alpha) \tag{4}$$

gegeben. Andere Interpolationsfunktionen sind möglich und gebräuchlich und können dem Verlauf des Wellenfrontfehlers angepasst werden. Unter der zirkulären Brechkraft ist jene Brechkraft zu verstehen, die eine rotationssymmetrische Linse aufweist, und deren Front- und Rückradien durch die Radien im betrachteten Meridian der torischen Linse gegeben sind. Dabei kann es sich um eine torische Linse mit einer torischen Fläche und einer rotationssymmetrischen Linse handeln, oder um eine torische Linse mit zwei torischen Linsenflächen.

Die Normalvektoren auf die torische Fläche bzw. Flächen einer torischen Linse sind zur Linsenachse windschief, ausgenommen in den Hauptmeridianen.

Fig. 2 zeigt das zirkuläre Brechkraftprofil der Linse gemäß Fig. 1. Aus Fig. 2 kann abgeleitet werden, daß ausschließlich in den Meridianwinkeln $\alpha$, in denen gilt

$$\frac{\partial D}{\partial \alpha} = 0 \qquad\qquad (5)$$

die Normalvektoren auf die Linsenflächen mit der Linsenachse eine Ebene aufspannen.

Die oben definierte Wiederholungsrate der Linse bzw. zumindest einer Linsenfläche der Linse gemäß Fig. 1 ist m = 2.

Durch die Gegebenheit, daß in den Hauptmeridianen die Normalvektoren auf die Linsenfläche bzw. Linsenflächen zur Linsenachse nicht windschief sind, lassen sich die Brechkräfte in diesen Hauptmeridianen z.B. mit einem Scheitelbrechwertmesser bestimmen. Weiters kann durch geeignete Geräte der Winkel zwischen den Hauptmeridianen bestimmt werden. Die Meridianbrechkräfte in Positionen zwischen den Hauptmeridianen lassen sich dagegen im allgemeinen nicht bestimmen.

Im Folgenden wird die Meridianbrechkraft in einem Meridian bzw. Halbmeridian der Linsenfläche, in dem der Nonnalvektor auf die Linsenfläche mit der Linsenachse eine Ebene aufspannt, "diskrete Brechkraft" genannt.

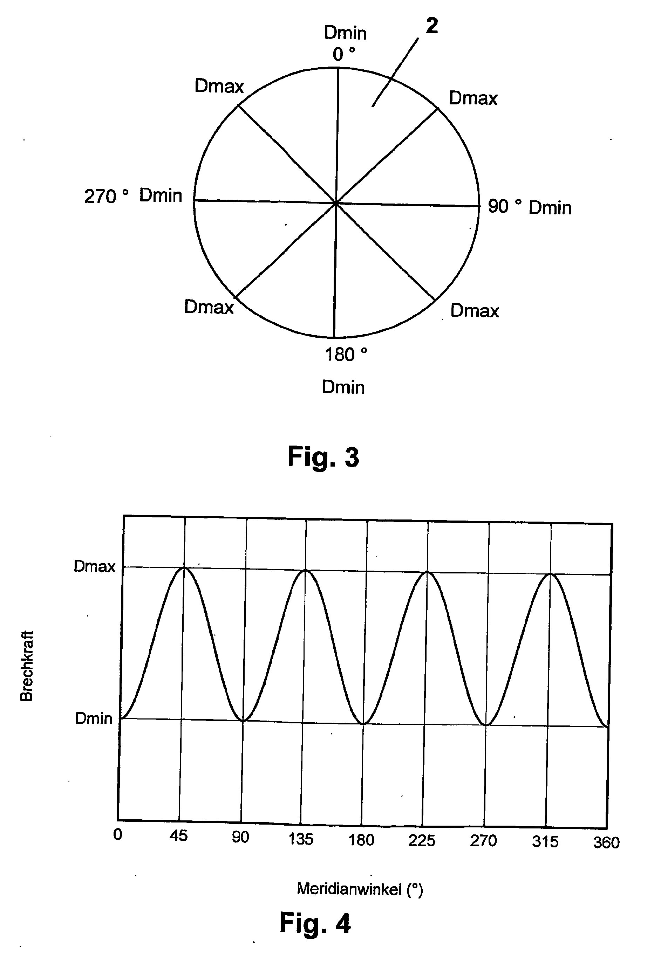

In Fig. 3 ist eine supertorische Linse 2 in Aufsicht dargestellt, bei der die Wiederholungsrate m = 4 ist. Das zirkuläre Brechkraftprofil der Linse ist in Fig. 4 dargestellt.

Die Linse gemäß Fig. 4 ist dazu geeignet, den Vierblattfehler einer Wellenfront zu kompensieren.

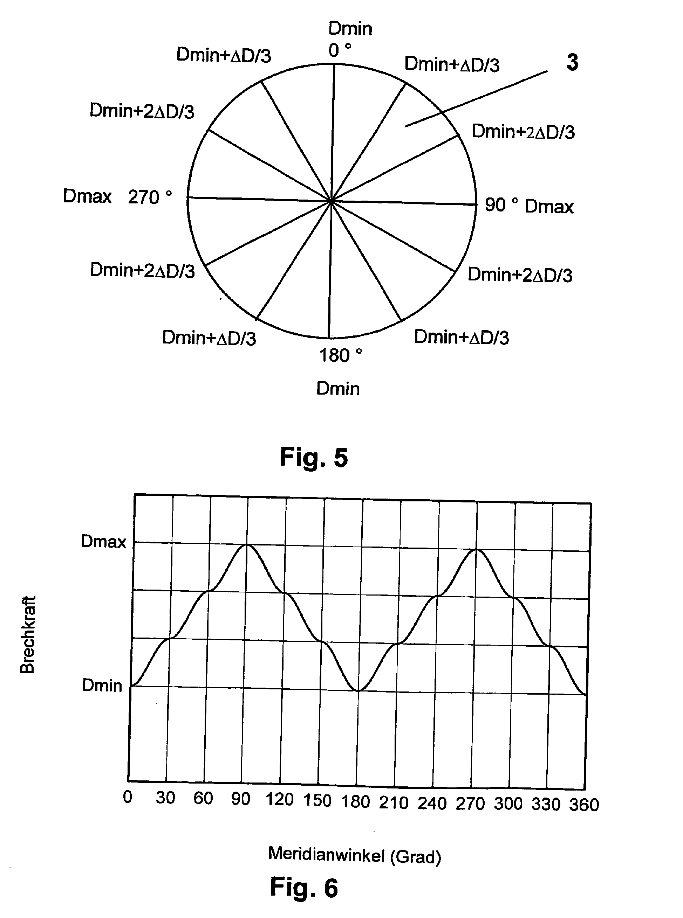

In Fig. 5 ist eine erfindungsgemäße diskret torische Linse 3 in Aufsicht dargestellt. Die Wiederholungsrate dieser Linse ist m = 2. Diese Linse unterscheidet sich von herkömmlichen torischen Linsen mit gleicher Wiederholungsrate dadurch, daß sie in 6 Meridianen bzw. 12 Halbmeridianen Flächenelemente aufweist, deren Normalvektoren mit der Linsenachse Ebenen aufspannen, also zur Linsenachse nicht windschief sind. Diese Linse besitzt somit in 6 Meridianen bzw. in 12 Halbmeridianen diskrete Brechkräfte.

Das zirkuläre Brechkraftprofil der Linse gemäß Fig. 5 ist in Fig. 6 dargestellt. Wie ersichtlich, hat die Linse in 6 Meridianen diskrete Brechkräfte. Somit ist die Linse multifokal und weist eine Tiefenschärfe auf, die größer ist als eine rotationssymmetrische Linse gleichen Durchmessers mit glatten Oberflächen.

Beträgt die minimale Brechkraft Dmin der Linse gemäß Fig. 5 z.B. 20 Dioptrien, und die maximale Brechkraft Dmax 23 Dioptrien, so besitzt diese Linse diskrete Brechkräfte von 20, 21, 22 und 23 Dioptrien.

Zur Beurteilung der Abbildungsqualität diskret torischer bzw. supertorischer Linsen dienen Abschätzungen der optischen Weglängenfehler in Defocus-Positionen:

Wie in "W. Fiala, J. Pingitzer. Analytical approach to diffractive multifocal lenses", Eur. Phy. J AP 9, 227-234 (2000)" ausgeführt, beträgt der optische Weglängenfehler PLE in einer Defocus-Position von $\Delta D$ Dioptrien:

$$PLE = \frac{\Delta D \times B^2}{8} \qquad\qquad (6)$$

In Gleichung 6 ist B der Durchmesser einer Linse.

Sind zwei diskrete Brechkräfte im Abstand von 1 Dioptrie vorhanden (wie im obigen Beispiel), so ist der mittlere Defocus $\Delta D_{av}$ gleich 0.5 Dioptrien. Damit ist der mittlere optische Weglängenfehler $PLE_{av}$ gegeben durch:

$$PLE_{av} = \frac{0.5 \times B^2}{8} \qquad\qquad (6')$$

Bei Diffraktionslinsen mit gleicher relativer Intensität in der nullten und ersten Diffraktrionsordnung beträgt der optische Weglängenfehler in beiden Brechkräften eine halbe Wellenlänge, also ca. 0.28 $\mu$m (siehe W. Fiala, J. Pingitzer, loc. cit.). Bekannterweise ist die Abbildungsqualität solcher bifokaler Linsen zufriedenstellend.

Läßt man deshalb einen Weglängenfehler $PLE_{av}$ = 0.28 $\mu$m zu, so ergibt sich nach Gleichung 6' ein Linsendurchmesser von 2.12 mm. Das bedeutet, daß die Linse gemäß Fig. 5 unter den obigen Annahmen bis zu einem Durchmesser von 2.12 eine kontinuierliche Tiefenschärfe von mindestens 3 Dioptrien aufweist, die Linse kann in diesem Bereich als "omnifokal" bezeichnet werden.

Bei kreisförmigen Linsen stellt sich Kontrastumkehr bei einem Weglängenfehler $PLE_{KU}$ von

$$PLE_{KU} = \frac{\lambda\sqrt{2}}{2} \qquad\qquad (7)$$

ein. Lässt man einen Weglängenfehler gemäß Gleichung 7 zu, so erhöht sich der zulässige Durchmesser der Linse auf 2.5 mm.

Damit ist gezeigt, daß diskret supertorische Linsen entsprechend der gegenständlichen Erfindung bei größeren Linsendurchmessern multifokal sind, und bei kleineren Durchmessern eine große Tiefenschärfe aufweisen, d.h. omnifokal sind.

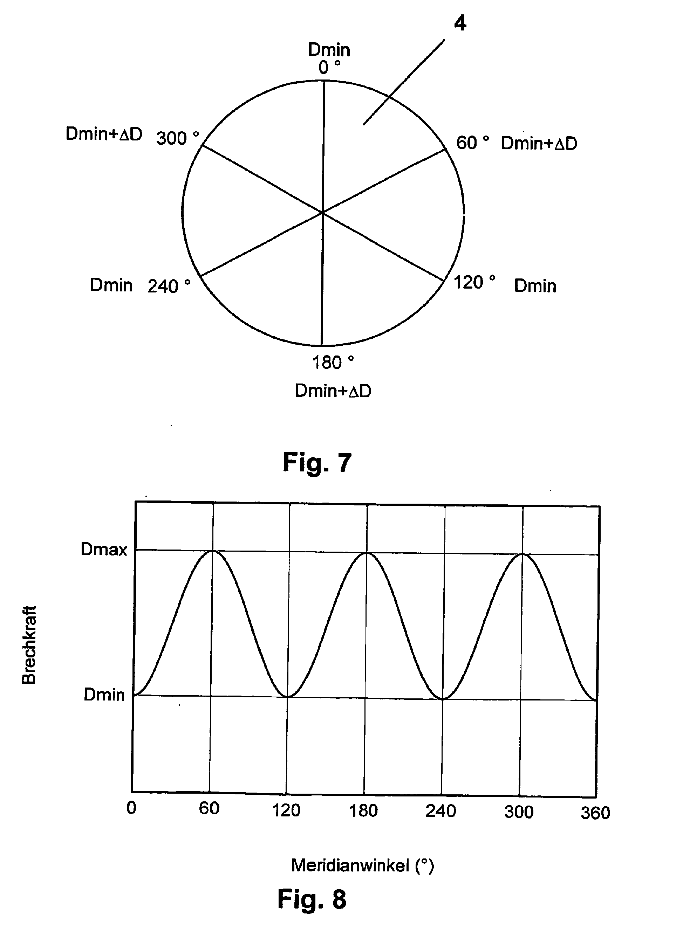

In Fig. 7 ist eine supertorische Linse 4 in Aufsicht dargestellt. Die Wiederholungsrate dieser Linse ist m = 3. Das zirkuläre Brechkraftprofil der Linse gemäß Fig. 7 ist in Fig. 8 dargestellt. Eine Linse gemäß Fig. 7 eignet sich zur Kompensation des Dreiblattfehlers einer Wellenfront.

Wie ersichtlich, weist eine Linse gemäß Fig. 7 nur in Halbmeridianen diskrete Brechkräfte auf. So weist die Linse gemäß Fig. 7 in den Halbmeridianen 0°, 120° und 240° die Brechkraft Dmin, und in den Halbmeridianen 60°, 180° und 300° die Brechkraft Dmax = Dmin + $\Delta$D auf.

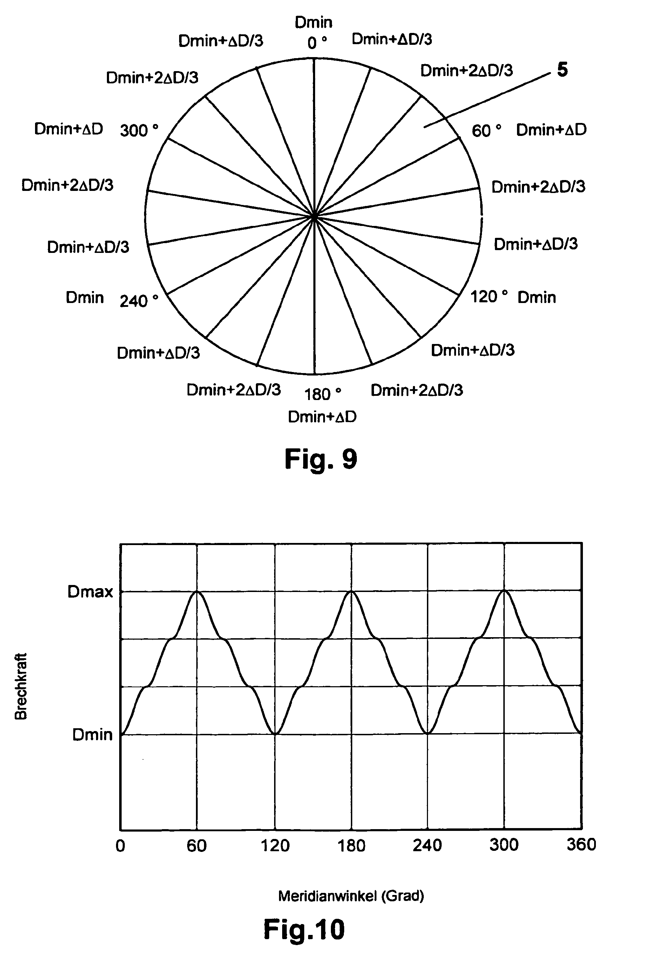

Fig. 9 zeigt eine diskret supertorische Linse 5 in Aufsicht. Die Wiederholungsrate dieser Linse beträgt m = 3. Die Linse weist in insgesamt 18 Halbmeridianen diskrete Brechkräfte auf. Das zirkuläre Brechkraftprofil der Linse gemäß Fig. 9 ist in Fig. 10 dargestellt. Die im Zusammenhang mit der Diskussion der Abbildungsqualität einer Linse gemäß Fig. 5 getroffenen Aussagen gelten für diese Linse sinngemäß. Die Linse ist bei großen Durchmessern multifokal, und bei kleinen Durchmessern omnifokal.

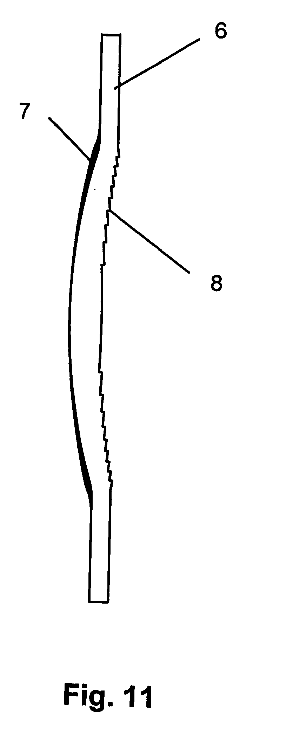

In Fig. 11 ist im Querschnitt eine weitere Linse 6 dargestellt. Die Linse besitzt eine Frontfläche 7 mit zirkulärem Brechkraftprofil, z.B. torisch, diskret torisch, supertorisch oder diskret supertorisch wie zuvor erörtert, und eine Rückfläche 8 mit radialem Brechkraftprofil, z.B. in annulare Zonen unterteilt und mit optischen Stufen zwischen den einzelnen annularen Zonen wie in US 5,982,543 (Fiala) bzw. US 7,287,852 B2 (Fiala) beschrieben.

Das zirkuläre und das radiale Brechkraftprofil können jeweils sowohl durch die Gestaltung der einen als auch der anderen Oberfläche 7, 8 und auch durch eine Kombination der Oberflächen 7, 8 gebildet sein.

Durch die Kombination von zirkulärem und radialem Brechkraftprofil wird erreicht, daß diese Linse auch bei großen Durchmessern eine große Tiefenschärfe besitzt, also auch bei großen Durchmessern die Eigenschaft besitzt, omnifokal zu sein.

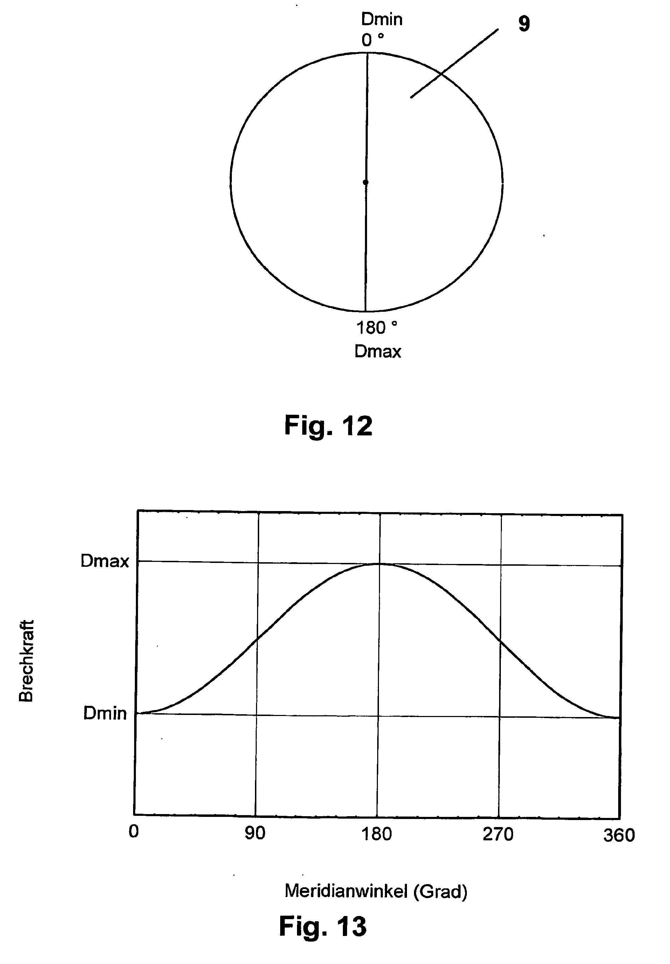

In Fig. 12 ist eine weitere Linse 9 in Aufsicht dargestellt. Das zirkuläre Brechkraftprofil der Linse gemäß Fig. 12 ist in Fig. 13 dargestellt.

Wie ersichtlich, ist die Wiederholungsrate der Linse gemäß Fig. 12 m = 1. Im Halbmeridian 0° besitzt die Linse eine diskrete Brechkraft Dmin, im Halbmeridian 180° besitzt die Linse eine diskrete Brechkraft von Dmax.

Linsen gemäß Fig. 12 eignen sich zur Korrektur des Wellenfrontfehlers Kippung und Koma.

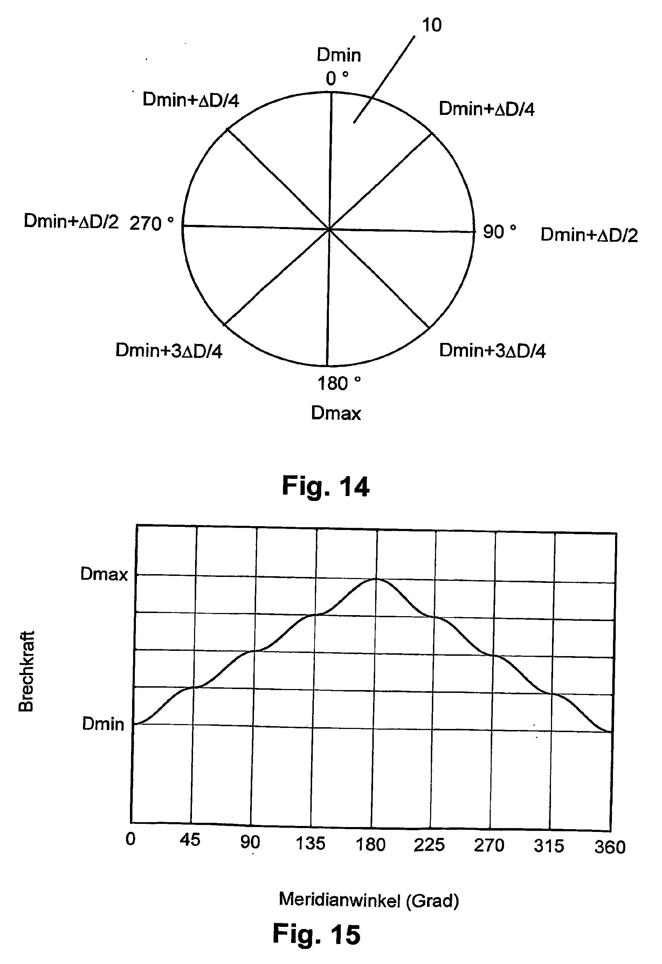

Schließlich ist in Fig. 14 eine diskret supertorische Linse 10 in Aufsicht dargestellt. Die Wiederholungsrate dieser Linse ist m = 1. Die Linse besitzt in 8 Halbmeridianen diskrete Brechkräfte. Das zirkuläre Brechkraftprofil der Linse gemäß Fig. 14 ist in Fig. 15 dargestellt.

Die Linse besitzt auch bei großem Durchmesser eine große Tiefenschärfe, wenn die Fläche einer Linse gemäß Fig. 14 mit einer in Zonen unterteilten Fläche 8 gemäß Fig. 11 kombiniert wird. Das im Zusammenhang mit der Linse gemäß Fig. 5 Gesagte gilt sinngemäß.

Linsen mit zirkulärem Brechkraftprofil gemäß der gegenständlichen Erfindung können mit modernen Linsendrehbänken, die zur Herstellung von Freiformflächen geeignet sind (z.B. EPT Optomatic, Fa. Rigeo, NL, oder Modell Optoform, Fa. Precitech, USA), erzeugt werden.

Die Erfindung ist nicht auf die dargestellten Ausführungsformen beschränkt, sondern umfaßt alle Varianten und Modifikationen, die in den Rahmen der angeschlossenen Ansprüche fallen.

## Patentansprüche

1. Linse mit zirkulärem Brechkraftprofil ($D(\alpha)$), **dadurch gekennzeichnet, daß** sie in zumindest einem Halbmeridian, der zwischen Halbmeridianen mit der minimalen und der maximalen Brechkraft ($D_{min}$, $D_{max}$) der Linse (3, 5, 6, 10) liegt, eine Brechkraft (D) aufweist, die zwischen der minimalen und der maximalen Brechkraft der Linse liegt und deren Ableitung ($\partial D/\partial \alpha$) nach dem Meridianwinkel ($\alpha$) gleich Null ist.

2. Linse nach Anspruch 1, **dadurch gekennzeichnet, daß** sie nur einen Halbmeridian mit der minimalen Brechkraft ($D_{min}$) und nur einen Halbmeridian mit der maximalen Brechkraft ($D_{max}$) der Linse (10) hat.

3. Linse nach Anspruch 1, **dadurch gekennzeichnet, daß** sie mehr als zwei Halbmeridiane mit der minimalen Brechkraft ($D_{min}$) und mehr als zwei Halbmeridiane mit der maximalen Brechkraft ($D_{max}$) der Linse (5) hat.

4. Linse nach einem der Ansprüche 1 bis 3, **dadurch gekennzeichnet, daß** die Wiederholungsrate (m) ihres zirkulären Brechkraftprofils ($D(\alpha)$) gleich 1 ist.

5. Linse nach einem der Ansprüche 1 bis 3, **dadurch gekennzeichnet, daß** die Wiederholungsrate (m) ihres zirkulären Brechkraftprofils ($D(\alpha)$) gleich 2 ist.

6. Linse nach einem der Ansprüche 1 bis 3, **dadurch gekennzeichnet, daß** die Wiederholungsrate (m) ihres zirkulären Brechkraftprofils ($D(\alpha)$) größer gleich 3 ist.

7. Linse nach einem der Ansprüche 1 bis 6, **dadurch gekennzeichnet, daß** sie zusätzlich mit einem radialen Brechkraftprofil versehen ist.

8. Linse nach Anspruch 7, **dadurch gekennzeichnet, daß** das zirkuläre Brechkraftprofil durch Gestaltung der einen und das radiale Brechkraftprofil durch Gestaltung der anderen Oberfläche (7, 8) der Linse (6) gebildet ist.

9. Linse nach Anspruch 7 oder 8, **dadurch gekennzeichnet, daß** das radiale Brechkraftprofil in an sich bekannter Weise durch annulare Zonen mit dazwischenliegenden optischen Stufen gebildet ist.

10. Linse nach einem der Ansprüche 1 bis 9, **dadurch gekennzeichnet, daß** sie eine Intraokularlinse ist.

11. Linse nach einem der Ansprüche 1 bis 9, **dadurch gekennzeichnet, daß** sie eine Kontaktlinse ist.

12. Linse nach einem der Ansprüche 1 bis 9, **dadurch gekennzeichnet, daß** sie eine Linse einer optischen Vorrichtung ist.

## Claims

1. A lens having a circular refractive power profile ($D(\alpha)$), **characterized in that** in at least one semi-meridian located between semi-meridians having the minimum and the maximum refractive power ($D_{min}$, $D_{max}$) of the lens (3, 5, 6, 10) it has a refractive power (D) that is between the minimum and the maximum refractive power of the lens and whose derivative ($\partial D/\partial \alpha$) with respect to the meridian angle ($\alpha$) is equal to zero.

2. The lens as claimed in claim 1, **characterized in that** it has only one semi-meridian having the minimum refractive power ($D_{min}$), and only one semi-meridian having the maximum refractive power ($D_{max}$), of the lens (10).

3. The lens as claimed in claim 1, **characterized in that** it has more than two semi-meridians having the minimum refractive power ($D_{min}$), and more than two semi-meridians having the maximum refractive power ($D_{max}$), of the lens (5).

**4.** The lens as claimed in one of claims 1 to 3, **characterized in that** the repetition rate (m) of its circular refractive power profile $(D(\alpha))$ is equal to 1.

**5.** The lens as claimed in one of claims 1 to 3, **characterized in that** the repetition rate (m) of its circular refractive power profile $(D(\alpha))$ is equal to 2.

**6.** The lens as claimed in one of claims 1 to 3, **characterized in that** the repetiton rate (m) of its circular refractive power profile $(D(\alpha))$ is greater than or equal to 3.

**7.** The lens as claimed in one of claims 1 to 6, **characterized in that** it is additionally provided with a radial refractive power profile.

**8.** The lens as claimed in claim 7, **characterized in that** the circular refractive power profile is formed by configuring one surface (7) of the lens (6), and the radial refractive power profile is formed by configuring the other surface (8) of the lens (6).

**9.** The lens as claimed in claim 7 or 8, **characterized in that** the radial refractive power profile is formed in a way known by per se by annular zones with optical stages situated therebetween.

**10.** The lens as claimed in one of claims 1 to 9, **characterized in that** it is an intraocular lens.

**11.** The lens as claimed in one of claims 1 to 9, **characterized in that** it is a contact lens.

**12.** The lens as claimed in one of claims 1 to 9, **characterized in that** it is a lens of an optical device.

**Revendications**

**1.** Lentille ayant un profil de puissance circulaire $(D(\alpha))$, **caractérisée en ce qu'**elle présente dans au moins un demi-méridien, qui est situé entre les demi-méridiens ayant la puissance minimale et la puissance maximale $(D_{min}, D_{max})$ de la lentille (3, 5, 6, 10), une puissance (D) qui est située entre la puissance minimale et la puissance maximale de la lentille et dont la dérivée $(\partial D/\partial\alpha)$ par rapport à l'angle des méridiens $(\alpha)$ est égale à zéro.

**2.** Lentille selon la revendication 1 **caractérisée en ce qu'**elle n'a qu'un demi-méridien ayant la puissance minimale $(D_{min})$ et qu'un demi-méridien ayant la puissance maximale $(D_{max})$ de la lentille (10).

**3.** Lentille selon la revendication 1 **caractérisée en ce qu'**elle a plus de deux demi-méridiens ayant la puissance minimale $(D_{min})$ et plus de deux demi-méridiens ayant la puissance maximale $(D_{max})$ de la lentille (5).

**4.** Lentille selon l'une des revendications 1 à 3 **caractérisée en ce que** le taux de répétition (m) de son profil de puissance circulaire $(D(\alpha))$ est égal à 1.

**5.** Lentille selon l'une des revendications 1 à 3 **caractérisée en ce que** le taux de répétition (m) de son profil de puissance circulaire $(D(\alpha))$ est égal à 2.

**6.** Lentille selon l'une des revendications 1 à 3 **caractérisée en ce que** le taux de répétition (m) de son profil de puissance circulaire $(D(\alpha))$ est supérieur ou égal à 3.

**7.** Lentille selon l'une des revendications 1 à 6 **caractérisée en ce qu'**elle est munie en outre d'un profil de puissance radial.

**8.** Lentille selon la revendication 7 **caractérisée en ce que** le profil de puissance circulaire est formé par façonnage d'une surface (7) de la lentille (6) et le profil de puissance radial est formé par façonnage de l'autre surface (8) de la lentille (6).

**9.** Lentille selon la revendication 7 ou 8 **caractérisée en ce que** le profil de puissance radial est formé de manière connue en soi par des zones annulaires avec des échelons optiques situés entre elles.

**10.** Lentille selon l'une des revendications 1 à 9 **caractérisée en ce que** c'est une lentille intraoculaire.

**11.** Lentille selon l'une des revendications 1 à 9 **caractérisée en ce que** c'est une lentille de contact.

**12.** Lentille selon l'une des revendications 1 à 9 **caractérisée en ce que** c'est une lentille d'un dispositif optique.

Dmin
0 °

1

Dmax 270 °                    90 ° Dmax

180 °
Dmin

## Fig. 1

Brechkraft

Dmax

ΔD

Dmin

0          90          180          270          360

Meridianwinkel (Grad)

## Fig. 2

Fig. 3

Meridianwinkel (°)

Fig. 4

**Fig. 5**

Meridianwinkel (Grad)

**Fig. 6**

**Fig. 7**

Meridianwinkel (°)

**Fig. 8**

**Fig. 9**

Meridianwinkel (Grad)

**Fig.10**

14

**Fig. 11**

Dmin
0 °

**9**

180 °
Dmax

## Fig. 12

Brechkraft

Dmax

Dmin

0    90    180    270    360

Meridianwinkel (Grad)

## Fig. 13

**Fig. 14**

Meridianwinkel (Grad)

**Fig. 15**

**IN DER BESCHREIBUNG AUFGEFÜHRTE DOKUMENTE**

**In der Beschreibung aufgeführte Patentdokumente**

- WO 2006136424 A1
- WO 2006236424 A1
- US 5982543 A, Fiala
- US 7287852 B2, Fiala

**In der Beschreibung aufgeführte Nicht-Patentliteratur**

- **W. FIALA ; J. PINGITZER.** Analytical approach to diffractive multifocal lenses. *Eur. Phy. J AP,* 2000, vol. 9, 227-234